(19) Europäisches Patentamt
European Patent Office
Office européen des brevets

(11) **EP 3 649 986 B1**

(12) **EUROPEAN PATENT SPECIFICATION**

(45) Date of publication and mention
of the grant of the patent:
**15.11.2023   Bulletin 2023/46**

(51) International Patent Classification (IPC):
**A61F 2/30** (2006.01)      **A61B 17/58** (2006.01)
**A61C 13/00** (2006.01)      **A61L 27/06** (2006.01)
**A61L 27/50** (2006.01)      **A61L 27/56** (2006.01)

(21) Application number: **18827933.5**

(22) Date of filing: **09.07.2018**

(52) Cooperative Patent Classification (CPC):
**A61C 8/0013; A61B 17/58; A61F 2/30; A61K 6/84;
A61L 27/06; A61L 27/50; A61L 27/56;
B23K 26/352; B23K 26/3584;** A61C 2008/0046;
B23K 2103/14

(86) International application number:
**PCT/JP2018/025800**

(87) International publication number:
**WO 2019/009428 (10.01.2019 Gazette 2019/02)**

(54) **IMPLANT AND METHOD FOR MANUFACTURING SAME**

IMPLANTAT UND VERFAHREN ZU SEINER HERSTELLUNG

IMPLANT ET SON PROCÉDÉ DE FABRICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.07.2017   JP 2017133498
15.11.2017   JP 2017219912
16.02.2018   JP 2018025671
19.06.2018   JP 2018115873**

(43) Date of publication of application:
**13.05.2020   Bulletin 2020/20**

(73) Proprietor: **Daicel Miraizu Ltd.**
**Tokyo 108-8231 (JP)**

(72) Inventors:
• **ITAKURA Masahiko**
**Tokyo 108-8231 (JP)**
• **SHIMIZU Kiyoshi**
**Tokyo 108-8231 (JP)**
• **UNO Takayuki**
**Tokyo 108-8231 (JP)**
• **KATAYAMA Masahiro**
**Tokyo 108-8231 (JP)**

(74) Representative: **Grünecker Patent- und
Rechtsanwälte
PartG mbB
Leopoldstraße 4
80802 München (DE)**

(56) References cited:
**WO-A1-2013/150369      DE-U1- 9 013 067
JP-A- S63 160 662      JP-A- 2016 043 413
JP-A- 2016 078 090**

• **Mizutani, Masayoshi et al.: "High
functionalization of Dental Implant with laser
fabrication", Journal of the Japan Society for
Precision Engineering, vol. 81, no. 12, 2015,
pages 1073-1077, XP055658963, ISSN: 1882-675x,
DOI: 10.2493/jjspe.81.1073**
• **Mizutani, Masayoshi: "Creation of bone
tissue-compatible implant by means of nano
pulse laser", Report of Grant-supported
researches, The Armada Foundation, vol. 27 ,
pages 158-163, ISSN: 2434-0723**
• **Asri, R.I.M. et al: "Corrosion and surface
modification on biocompatible metals: A review",
Materials Science and Engineering C, vol. 77,
August 2017 (2017-08), pages 1261-1274,
XP085028532, ISSN: 0928-4931, DOI:
10.1016/j.msec.2017.04.102**

**Description**

Technical Field

The present invention relates to an implant suitable for binding to a biological tissue, and a method for manufacturing the implant.

Background Art

An implants made from titanium or a titanium alloy is known as an implant for binding to a biological tissue. An implant binds to a biological tissue, such as bone or teeth, and exists in a living body, hence the implant is required to have both bindability to bone or teeth as well as strength. Thus, an implant is known that has, from such a viewpoint, a porous structure at a site of binding to a biological tissue, as described in documents below.

JP 5920030 B describes an invention of a porous implant material, and the use of a porous metal body having a three-dimensional network structure which is formed from a continuous skeleton and in which a plurality of pores are interconnected, and indicates that the porous metal body is produced by forming a foamed slurry containing metal powder and an blowing agent, and blowing and sintering the foamed slurry (claim 1, paragraph 0013).

JP 2009-254581 A describes an invention of a biological implant, and indicates that the biological implant includes a surface portion that is a site of binding to a biological tissue and a core portion provided inside the surface portion, and that the surface portion is made of a porous sintered body made of a metal and having vacancies formed therein (claim 1).

JP 2014-161520 A describes an invention of an implant and a method for manufacturing the implant, and indicates that shot blasting and electrolytic treatment are performed on a titanium-based base material (claim 1).

JP 5326164 B describes an invention of a biomaterial and a method for fabricating the biomaterial, and indicates that a biomaterial having a porous structure is manufactured by using as a mold a molded body made of a thin plate laminate having a porous structure (claim 1, paragraphs 0025, 0026).

Non-Patent Document Discovery of Osseous Tissue Compatible Implant Using Nanopulse Laser (Masayoshi Mizutani, Associate Professor, Graduate School of Engineering, Tohoku University, 2011, General Research and Development Grant AF-2011212) describes a technique for creating an osseous tissue compatible implant by using a nanopulse laser.

WO2013150369 A1 discloses a dental implant comprising in a part a trabecular structure with an external conformation which allows the best adhesion and integration possible of the bone,

Summary of Invention

An object of the present invention is to provide an implant that can be used in a biological tissue and a method for manufacturing the implant.

The present invention, according to claim 1, provides an implant used for binding to a biological tissue including bone or teeth, and made of metal selected from titanium or titanium alloys, cobalt chrome alloys, and tantalum, the implant including a surface layer portion of a portion, which is bound to a biological tissue including bone or teeth, of the implant, the surface layer portion having a porous structure. The porous structure includes a trunk hole formed in a thickness direction and including an opening on a binding face side, open holes each constituted of a branch hole formed extending from an inner wall surface of the trunk hole in a direction different from that of the trunk hole, an interior space formed in the thickness direction and not including an opening on the binding face side, a tunnel connecting path connecting the open holes and the interior space, and a tunnel connecting path connecting the open holes.

Further, the present invention provides a method for manufacturing the implant described above, according to claim 3.

Preferred embodiments of the invention are defined in the dependent claims.

The implant according to the present invention has better bindability to a biological tissue, including bone or teeth, due to the presence of the porous structure of the surface layer portion.

Brief Description of Drawings

FIGS. 1A and 1B are cross-sectional views illustrating examples of porous structures of an implant surface layer portion of the present invention.
FIGS. 2A to 2C are cross-sectional views illustrating examples of porous structures of an implant surface layer portion of the present invention that differs from that in FIGS. 1A and 1B.

FIG. 3 is an explanatory view of an embodiment of laser beam irradiation in a method for manufacturing an implant according to the present invention.

FIG. 4 is an explanatory view of an embodiment of laser beam irradiation in the method for manufacturing an implant according to the present invention.

FIG. 5 is a SEM image (magnification of 129) of a plate surface of pure titanium after laser beam irradiation in Example 1.

FIG. 6 is a SEM image (magnification of 137) of a plate surface of pure titanium after laser beam irradiation in Example 2.

FIG. 7 is a SEM image (magnification of 110) of a plate surface of 64 titanium after laser beam irradiation in Example 3.

FIG. 8 is a SEM image of a plate surface of pure titanium after laser beam irradiation in Example 5.

FIG. 9 is a SEM image of a plate surface of pure titanium after laser beam irradiation in Example 7.

FIG. 10 is a SEM image of a plate surface of pure titanium after laser beam irradiation in Example 8.

FIG. 11 is a SEM image of a plate surface of 64 titanium after laser beam irradiation in Example 9.

FIG. 12 is a SEM image of a plate surface of 64 titanium after laser beam irradiation in Example 11.

FIG. 13 is a SEM image of a plate surface of 64 titanium after laser beam irradiation in Example 12.

FIGS. 14A and 14B are x-ray CT scan images of a plate surface of 64 titanium after laser beam irradiation in Example 13, with FIG. 14A being a cross sectional image parallel to a scanning direction of the laser beam, and FIG. 14B being a cross-sectional image perpendicular to the scanning direction of the laser beam.

FIG. 15 is a SEM image of a plate surface of tantalum after laser beam irradiation in Example 14.

FIG. 16 is a SEM image of a plate surface of tantalum after laser beam irradiation in Example 15.

FIG. 17 is a SEM image of a plate surface of tantalum after laser beam irradiation in Example 16.

FIG. 18 is a SEM image of a plate surface of tantalum after laser beam irradiation in Example 17.

FIG. 19 is a SEM image of a plate surface of tantalum after laser beam irradiation in Example 18.

Description of Embodiments

Implant

An implant according to the present invention is used for binding to a biological tissue including bone or teeth. Examples of the implant include prosthetic joints, such as a prosthetic hip (stem, cup) and a prosthetic knee, and also an implant for fracture fixation (plate, screw), prosthetic tooth roots, and the like.

The implant according to the present invention is composed of metal selected from titanium (pure titanium), titanium alloys, cobalt chrome alloys, and tantalum. The titanium alloys and cobalt chrome alloys are those used as medical (including dental) titanium alloys and cobalt chrome alloys. For example, as the cobalt chrome alloy, Aichrom (available from ids Co., Ltd.), Premier Cast Hard (available from Denken-HighDental Co., Ltd.), and the like can be used.

The implant includes a surface layer portion, which has a porous structure, at a portion bound to a biological tissue, including bone or teeth, in the implant.

The porous structure includes a trunk hole formed in a thickness direction and including an opening on the coupling face side, open holes, each constituted of a branch hole formed extending from an inner wall surface of the trunk hole in a direction different from that of the trunk hole, and an interior space formed in the thickness direction and not including an opening on the coupling face side, and moreover the porous structure also includes a tunnel connecting path connecting the open holes and the interior space, and a tunnel connecting path connecting the open holes.

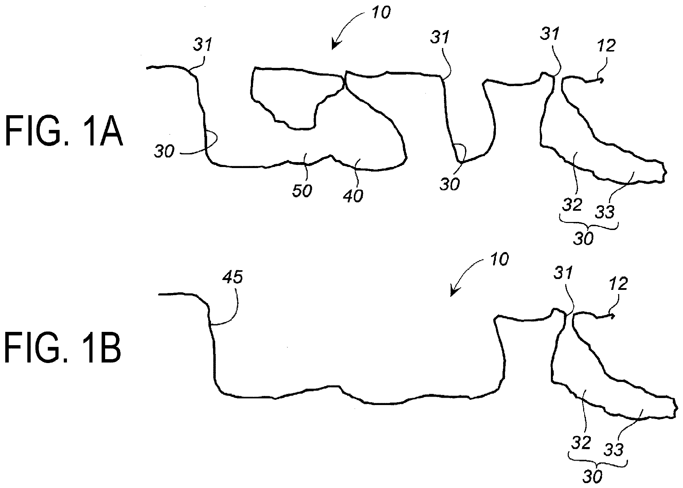

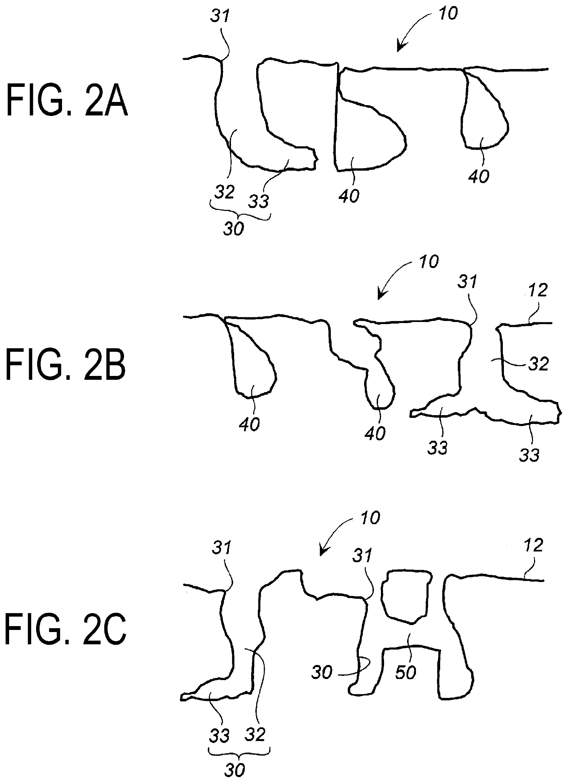

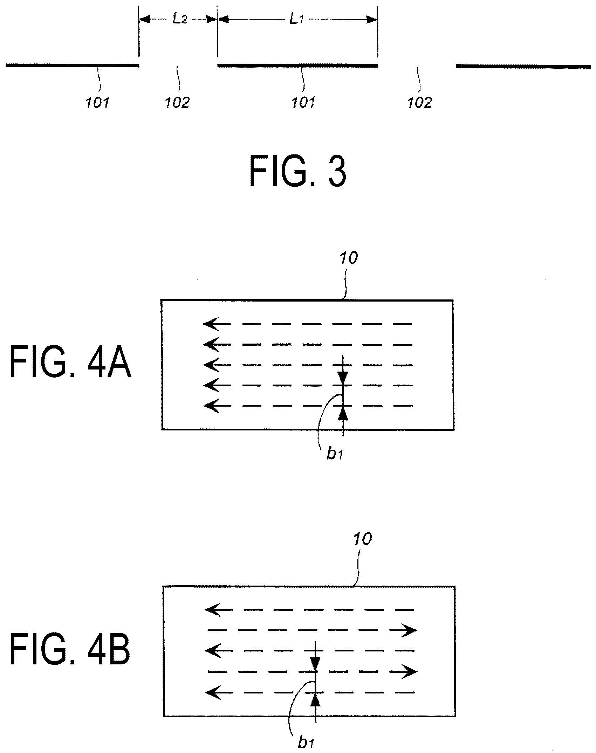

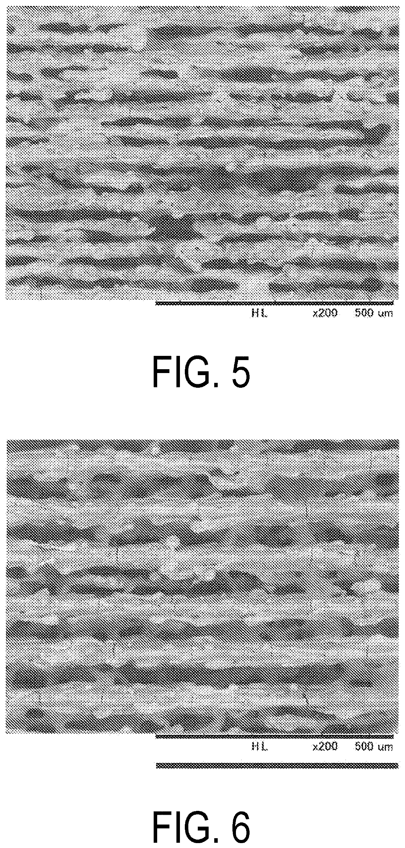

The porous structure of the surface layer portion of the implant is, for example, a porous structure such as illustrated in FIGS. 1A and 1B and FIGS. 2A to 2C, and is the same as the porous structure illustrated in FIG. 4 and FIG. 5 of JP 5860190 B.

The surface layer portion of an implant 10 includes an open hole 30 having an opening 31 on a binding face 12 side that binds to a biological tissue. The open hole 30 includes a trunk hole 32 having the opening 31 formed in a thickness direction, and a branch hole 33 formed from an inner wall surface of the trunk hole 32 in a direction different from a direction, in which the trunk hole 32 is formed. One or a plurality of the branch holes 33 may be formed.

Further, the surface layer portion of the implant 10 includes, on the binding face 12 side that binds to a biological tissue, an interior space 40 not having an opening. The interior space 40 is connected to the open hole 30 by a tunnel connecting path 50.

Further, the surface layer portion of the implant 10 may include an open space 45 in which a plurality of the open holes 30 become one hole, or the open space 45 may be formed by integration of the open hole 30 and the interior space 40 as one hole. One open space 45 has a greater internal volume than one open hole 30. Note that a multiplicity of the open holes 30 may be integrated to form the open space 45 having a groove shape.

Further, although not illustrated, the open hole 30 and the interior space 40 such as illustrated in FIGS. 2A and 2B may be connected by the tunnel connecting path 50, and the open holes 30 may be connected to each other by the tunnel connecting path 50, as illustrated in FIG. 2C.

Further, the interior spaces 40 such as illustrated in FIG. 2A may be connected to each other by the tunnel connecting path 50.

The surface layer portion, which has a porous structure, of the implant preferably has a depth ranging from 10 to 1000 $\mu$m from the surface to a depth of the open hole.

When the implant, which has a porous structure in the surface layer portion thereof, of the present invention is connected so as to be embedded in a bone, first, calcium phosphates contained in the body fluid in a supersaturated state precipitate and, at the same time, osteoblasts sense a space and thus are activated, whereby bone components are produced on both the bone and the implant, as described in the left column on P. 158 in Non-Patent Document. Eventually, the new bone completely fills the space between the bone and the implant (the porous structure of the implant surface layer portion), and a state of solid and dense adhesion is obtained.

Because the porous structure formed in the surface layer portion of the implant of the present invention is a complex structure as illustrated in FIG. 1 and FIG. 2, it is expected that this complex porous structure acts to increase the bindability between the bone and the implant.

Method for Manufacturing Implant

Next, a method for manufacturing an implant according to the present invention will be described. In forming a porous structure on the surface layer portion of the implant during manufacture of the implant, a surface including the surface layer portion is irradiated with a laser beam thereby forming a porous structure.

As a method for irradiating a laser beam, either one of laser beam irradiation methods below can be used:

(I) a method for continuously irradiating the surface, which is to become a portion of binding of the implant to a biological tissue, with a laser beam to form a straight line, a curved line, or a combination of the straight line and the curved line (first laser beam irradiation method), and

(II) a method for irradiating the surface, which is to become a portion of binding of the implant to the biological tissue, with a laser beam to alternately generate an irradiation portion irradiated by and a non-irradiation portion not irradiated by the laser beam when irradiating the laser beam to form a straight line, a curved line, or a combination of the straight line and the curved line (second laser beam irradiation method).

First Laser Beam Irradiation Method

The first laser beam irradiation method is known, and can be implemented in the same manner as methods for continuous irradiation with a laser beam described in JP 5774246 B, JP 5701414 B, JP 5860190 B, JP 5890054 B, JP 5959689 B, JP 2016-43413 A, JP 2016-36884 A, and JP 2016-44337 A.

The energy density needs to be 1 MW/cm$^2$ or greater. The energy density at the time of irradiation with a laser beam is determined from output power (W) of the laser beam and spot area (cm$^2$) ($\pi \cdot$[(spot diameter)/2]$^2$) of the laser beam. The energy density at the time of irradiation with a laser beam is preferably from 2 to 1000 MW/cm$^2$, more preferably from 10 to 800 MW/cm$^2$, and still more preferably from 10 to 700 MW/cm$^2$. The energy density can be adjusted to be within the ranges described above by increasing or decreasing the power of the laser beam or by increasing or decreasing the spot diameter of the laser beam.

The output power of the laser beam is preferably from 4 to 4000 W, more preferably from 50 to 2500 W, still more preferably from 150 to 2000 W, and even more preferably from 150 to 1000 W. However, the output power of the laser beam is preferably adjusted within the above described ranges in combination with the spot diameter to ensure the energy densities described above.

The beam diameter (spot diameter) is preferably from 5 to 80 $\mu$m. However, it is preferable to adjust the beam diameter within the above described ranges in combination with the output power of the laser beam to ensure the energy densities described above.

The irradiation rate of the laser beam is preferably from 2000 to 20000 mm/sec, more preferably from 2000 to 18000 mm/sec, and still more preferably from 3000 to 15000 mm/sec. The wavelength is preferably from 500 to 11000 nm.

The defocus distance is preferably from -5 to +5 mm, more preferably from -1 to +1 mm, and still more preferably from -0.5 to +0.1 mm. Laser irradiation may be performed with the defocus distance set to a constant value, or may be performed while changing the defocus distance. For example, when laser irradiation is performed, the defocus distance may be set to decrease, may be set to increase, or may be set to periodically increase and decrease.

The number of repetitions (a total number of times that irradiation with the laser beam is performed to form a single hole) is preferably from 1 to 50, more preferably from 5 to 30, and still more preferably from 5 to 20.

Second Laser Beam Irradiation Method

In the second laser beam irradiation method, performing irradiation to alternately generate an irradiation portion irradiated by and a non-irradiation portion not irradiated by the laser beam includes an embodiment of irradiation as illustrated in FIG. 3.

FIG. 3 illustrates a state in which a non-irradiation portion 102 not irradiated by the laser beam is alternately generated between an irradiation portion 101 irradiated by the laser beam and an adjacent irradiation portion 101 irradiated by the laser beam, resulting in forming a dotted line as a whole. At this time, the same portion can be repeatedly irradiated to form a line, which looks like one dotted line visually, as illustrated in FIG. 3. The number of repetitions can be from 1 to 20 times, for example.

When irradiation is performed a plurality of times, same irradiation portions may be irradiated with the laser beam, or different irradiation portions may be irradiated with the laser beam (the portions to be irradiated with the laser beam may be shifted) thereby roughening an entire metal piece. When irradiation is performed a plurality of times on same portions, the irradiation is implemented in a dotted line form. However, when irradiation is repeatedly performed while shifting the irradiation portions, i.e., shifting the irradiation portions to ensure that the irradiation portion irradiated by the laser beam overlaps a portion that was initially a non-irradiation portion not irradiated by the laser beam, irradiation is implemented eventually in a solid line state, even when irradiation is implemented a dotted line form.

When a metal molded body is continuously irradiated with a laser beam, the temperature of the irradiated surface increases. Hence, with a molded body having a small thickness, this may lead to deformation, such as warping, and thus a countermeasure such as cooling may be required. However, when laser irradiation is performed in a dotted line form as illustrated in FIG. 3, an irradiation portion 101 irradiated by the laser beam and a non-irradiation portion 102 not irradiated by the laser beam are alternately generated, hence the non-irradiation portion 102 not irradiated by the laser beam is cooled. Therefore, when continuous irradiation with a laser beam is performed, deformation, such as warping, does not readily occur even in a molded body having a small thickness, and thus this laser irradiation is preferred. At this time, even when different irradiation portions are irradiated by the laser beam (irradiation portions irradiated by the laser beam are shifted) as described above, the portions are irradiated by the laser beam in a dotted line form during irradiation, and thus similar effects can be achieved.

As the method of irradiation with a laser beam, a method for irradiating the surface of the implant 110 in one direction as illustrated in FIG. 4A or a method for irradiating the surface of the implant 110 from both directions, as in the dotted line illustrated in FIG. 4B, may be used. When irradiation with a laser beam is performed as illustrated in FIG. 4A and FIG. 4B, the irradiation portion irradiated with the laser beam can also be shifted as described above to form a solid line.

Additionally, the method may be a method for performing irradiation that makes dotted line irradiation portions, which are irradiated with the laser beam, intersect. The angle of intersection at this time is not particularly limited, but may be, for example, within a range from 45° to 90°. Further, using the method described above, irradiation may also be performed to make solid lines intersect. When irradiation is performed with laser beams intersecting, irradiation may be performed alternately in the intersecting directions, or may be performed a plurality of times in one direction only and subsequently a plurality of times in the intersecting directions. When irradiation is performed in the intersecting directions, irradiation may be performed the same number of times or a different number of times for each of the intersecting directions.

An interval b1 between each dotted line after irradiation can be adjusted in accordance with, for instance, area of the metal molded body to be irradiated, but may be, for example, within a range from 0.01 to 5 mm.

A length (L1) of the irradiation portion 101 irradiated by the laser beam and a length (L2) of the non-irradiation portion 102 not irradiated with the laser beam in FIG. 3 can be adjusted to be within a range of L1/L2 = 1/9 to 9/1. The length (L1) of the irradiation portion 101 irradiated with the laser beam is preferably 0.05 mm or greater, more preferably from 0.1 to 10 mm, and still more preferably from 0.3 to 7 mm to roughen the surface into a complex porous structure.

The second laser beam irradiation method can be implemented using

a method for performing irradiation with a laser beam using a combination of a galvano mirror and a galvano controller to pulse, by the galvano controller, a laser beam continuously oscillated from a laser oscillator thereby alternately generating an irradiation portion and a non-irradiation portion, or
a method performing irradiation with a laser beam using a fiber laser device provided with a direct-modulation modulator that directly converts a drive current of a laser and that is connected to a laser power supply thereby alternately generating an irradiation portion and a non-irradiation portion.

There are two types of laser excitation: pulsed excitation and continuous excitation, and pulsed wave lasers that are pulsed through pulsed excitation are commonly referred to as normal pulses.

A pulsed wave laser can be produced even with continuous excitation. The pulsed wave laser can be produced by: a Q-switched pulse oscillation method that makes a pulse width (pulse ON time) shorter than a normal pulse, thereby oscillating a laser having a higher peak power; an external modulation system that generates a pulsed wave laser by extracting light in time domain using an AOM or LN light intensity modulator; and a direct modulation system that directly modulates a laser drive current to produce a pulsed wave laser.

In the second laser beam irradiation method, the laser differs from the continuous wave laser used in the first laser irradiation method, but the energy density, irradiation rate of the laser beam, output power of the laser beam, wavelength, beam diameter (spot diameter), and defocus distance can be implemented similarly to those in the first laser irradiation method.

In the second laser beam irradiation method, when irradiation with a laser beam is performed to alternately generate an irradiation portion irradiated by and a non-irradiation portion not irradiated by the laser beam, irradiation is performed after adjusting duty ratio adjustment. The duty ratio is a ratio determined by the following equation from the ON time and OFF time of the laser beam output.

$$\text{Duty Ratio (\%)} = (\text{ON time})/(\text{ON time} + \text{OFF time}) \times 100$$

The duty ratio corresponds to L1/(L1 + L2) based on L1 (length of an irradiation portion irradiated with the laser beam) and L2 (length of a non-irradiation portion not irradiated with the laser beam) illustrated in FIG. 3, and can be selected from within a range from 10 to 90%.

By irradiating with the laser beam, with the duty ratio being adjusted, the laser beam can be irradiated in a dotted line form such as illustrated in FIG. 1. When the duty ratio is large, the efficiency in surface roughening improves, but the cooling effect deteriorates, while when the duty ratio is small, the cooling effect improves, but the surface roughening efficiency becomes poor. Hence. the duty ratio is adjusted according to a purpose.

The length (L1) of the irradiation portion 101 irradiated by the laser beam and the length (L2) of the non-irradiation portion 102 not irradiated by the laser beam can be adjusted to be within a range of L1/L2 = 1/9 to 9/1. The length (L1) of the irradiation portion 101 irradiated by the laser beam is preferably 0.05 mm or greater, more preferably from 0.1 to 10 mm, and still more preferably from 0.3 to 7 mm to roughen the surface into a complex porous structure.

A known laser can be used as the laser used in the first laser irradiation method and the second laser irradiation method, and for example, a $YVO_4$ laser, a fiber laser (single-mode fiber laser and multi-mode fiber laser), an excimer laser, a carbon dioxide laser, a UV laser, a YAG laser, a semiconductor laser, a glass laser, a ruby laser, a He-Ne laser, a nitrogen laser, a chelate laser, or a dye laser can be used.

In a case where the first laser beam irradiation method or the second laser beam irradiation method is performed in roughening the surface by irradiating with the laser beam, when a metal molded body is irradiated with a laser beam satisfying the energy density and the irradiation rate described above, the surface of the metal molded body is partially melted and evaporated, and thus the porous structure (FIG. 1 and FIG. 2) having a complex structure is formed.

When irradiation with a laser beam is performed in the manufacturing method according to the present invention,

(i) a method of bringing a non-irradiated surface of the implant not irradiated by the laser beam into contact with a substrate (for example, a steel plate, a copper plate, or an aluminum plate) made from a material having a thermal conductivity greater than or equal to that of metal selected from titanium or a titanium alloy, a cobalt chrome alloy, and tantalum that constitutes the implant (a material having a thermal conductivity of at least 100 W/m·k), or
(ii) a method of bringing a non-irradiated surface of the implant not irradiated by the laser beam into contact with a substrate (for example, a glass plate) made from a material having a thermal conductivity less than that of metal selected from titanium or a titanium alloy, a cobalt chrome alloy, or tantalum constituting the implant can be used.

As the method of (i), the method described in JP 2016-78090 A can be adopted and, as the method of (ii), the method described in JP 2016-124024 can be adopted.

The method of (i) can suppress an increase in temperature by dissipating heat generated when irradiating the implant made of metal, selected from titanium or a titanium alloy, a cobalt chrome alloy, and tantalum, with the laser beam.

The method of (ii) can suppress the dissipation of heat generated when irradiating the implant made of metal, selected from titanium or a titanium alloy, a cobalt chrome alloy, and tantalum, with the laser beam.

Therefore, when the method of (i) is implemented, changes in the size, depth, and shape of the holes can be suppressed, and when the method of (ii) is implemented, changes in the size, depth, and shape of the holes can be facilitated. Thus, the size, depth, and shape of the holes can be adjusted by selectively using the method of (i) or the method of (ii), depending on requirements.

When performing irradiation with a laser beam in the manufacturing method of the present invention, the laser beam can be irradiated while supplying an assist gas selected from air, oxygen, nitrogen, argon, and helium.

Irradiation with the laser beam while supplying the assist gas makes it possible to assist controlling the depth, size, and orientation (orientation of hole openings) of the holes, and also makes it possible to suppress the production of carbonized products and control surface properties. For example, when argon gas is selected, oxidation of the surface can be prevented, when oxygen gas is selected, oxidation of the surface can be promoted, and when nitrogen gas is selected, oxidation can be prevented and surface hardness can be improved.

When performing the first laser irradiation method or the second laser irradiation method, it is preferable to control the orientation of the holes (orientation of hole openings), the size of the holes, and the depth of the holes by adjusting the following requirements (a) to (g) or all of the requirements (a) to (h).

(a) Irradiation direction and angle of laser beam

Fixing of the irradiation direction of the laser beam to a specific direction and at a specific angle makes it possible to impart an orientation to the holes formed. The irradiation angle is preferably from 45 to 90 degrees with respect to the surface (implant surface) irradiated with the laser beam.

(b) Irradiation rate of the laser beam

The irradiation rate of the laser beam is preferably from 2000 to 20000 mm/sec, more preferably from 2000 to 18000 mm/sec, still more preferably from 2000 to 15000 mm/sec, and even more preferably from 3000 to 15000 mm/sec.

(c) Energy density when irradiating with laser beam

The energy density is preferably 1 MW/cm$^2$ or greater. The energy density at the time of irradiation with a laser beam is determined from output power (W) of the laser beam and spot area (cm$^2$) ($\pi \bullet$[(spot diameter)/2]$^2$) of the laser beam. The energy density at the time of irradiation with a laser beam is preferably from 2 to 1000 MW/cm$^2$, more preferably from 10 to 800 MW/cm$^2$, still more preferably from 50 to 700 MW/cm$^2$, even more preferably from 100 to 500 MW/cm$^2$, and still even more preferably from 100 to 300 MW/cm$^2$. As the energy density is increased, the holes become deeper and larger.

(d) Number of repetitions when irradiating with laser beam

The number of repetitions (a total number of times that irradiation with a laser beam is performed to form a single line) is preferably from 1 to 40, still preferably from 5 to 30, and even more preferably from 5 to 20. In a case where the same laser irradiation conditions are used for each repetition, as the number of repetitions increases, accordingly the holes (grooves) become deeper and larger along the line, and as the number of repetitions is reduced, accordingly the holes (grooves) become shallower and smaller along the line.

(e) Defocus distance of laser beam

The defocus distance is preferably from -5 to +5 mm, more preferably from -1 to +1 mm, and still more preferably from -0.5 to +0.1 mm. Laser irradiation may be performed with the defocus distance set to a constant value, or may be performed while changing the defocus distance. For example, when laser irradiation is performed, the defocus distance may be set to decrease, may be set to increase, or may be set to periodically increase and decrease. When the defocus distance is negative (-) (when focused on the inside of the metal molded body surface), the hole is deep and large. When the holes are to become deeper and larger, the defocus distance is preferably from -1 to +0.5 mm, more preferably from -0.5 to -0.05 mm, and still more preferably from -0.3 to -0.05 mm.

(f) Relationship of thermal conductivity between implant and substrate on which the implant is placed when irradiated with laser beam

As described above, the hole structure and the like can be adjusted by selection between a method for placing the implant on a substrate having a large thermal conductivity and a method of placing the implant on a substrate having a small thermal conductivity. As one example, the thermal conductivity relationship may be: thermal conductivity of implant < thermal conductivity of substrate.

(g) Line spacing of laser beam

The line spacing of the laser beam is the b1 interval in FIG. 4A or 4B. The laser beam line spacing is preferably from 0.01 to 3 mm, more preferably from 0.01 to 1 mm, still more preferably from 0.03 to 0.5 mm, and even more preferably 0.03 to 0.1 mm. All line spacings may be the same, or some or all of the line spacings may be different from one another.

A narrow line spacing between lines has a thermal impact on adjacent lines, and therefore the holes become large, the shape of the holes becomes more complex, and the depth of the holes tends to become deeper, and if the thermal impact is too great, a proper hole shape may not be formed. When the line spacing is wide, the holes become smaller, the shape of the holes does not become complex, and the holes do not tend to be very deep, but treatment speed can be enhanced.

(h) Duty ratio

The duty ratio is preferably from 10 to 90%, and more preferably from 30 to 80%.

Other

The output power of the laser beam is preferably from 4 to 4000 W, more preferably from 50 to 2000 W, still more preferably from 150 to 1000 W, even more preferably from 150 to 500 W, and still even more preferably from 150 to 300 W. In a case where other irradiation conditions of the laser beam are the same, as the output power increases, accordingly the holes become deeper and larger, and as the output power decreases, accordingly the holes become shallower and smaller. The wavelength is preferably from 500 to 11000 nm.

The respective ranges of the requirements (a) to (g) or the requirements (a) to (h), when performing the first laser irradiation method or the second laser irradiation method described above, may be combined as desired to control the orientation of the holes (orientation of hole openings), the size of the holes, and the depth of the holes.

Examples

Examples 1 and 2

Using the laser device described below, each of plates (length: 30 mm, width: 30 mm, thickness: 3 mm) of pure titanium was continuously irradiated in area of 20 mm x 6 mm under conditions shown in Table 1.

Laser Device

Oscillator: IPG-Yb fiber; YLR-300-SM
Galvano mirror SQUIREEL (available from by ARGES)
Light Focusing System: fc = 80 mm/f$\theta$ = 100 mm
Surface images (SEM images) of the plates of pure titanium after irradiation with a laser beam are presented in FIG. 5 (Example 1) and FIG. 6 (Example 2).

Example 3

Using the laser device described below, a plate (length: 30 mm, width: 30 mm, thickness: 1.5 mm) of 64 titanium was continuously irradiated with a laser beam in area of 20 mm x 6 mm under conditions shown in Table 1.

Laser Device

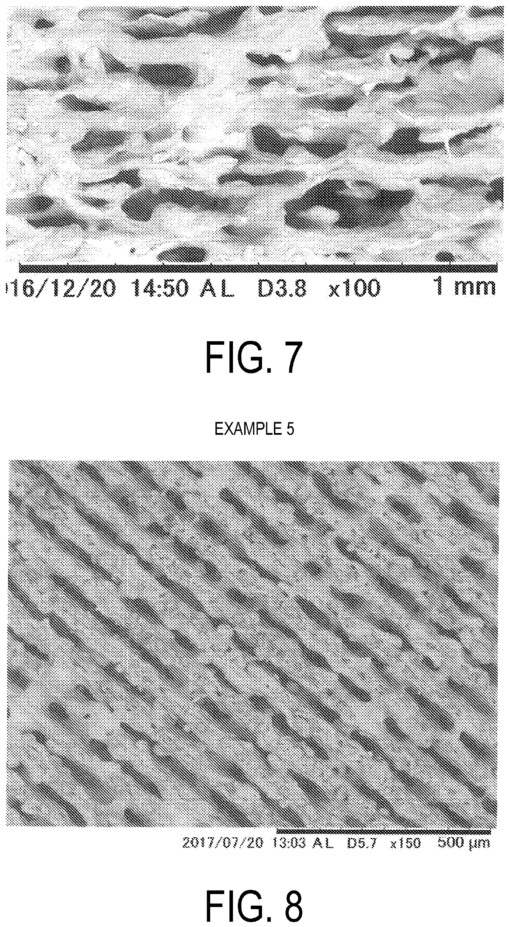

Oscillator: IPG-Yb fiber; YLR-300-SMAC
Galvano mirror SQUIREEL 16 (available from ARGES)
Light Focusing System: fc = 80 mm/f$\theta$ = 100 mm
A surface image (SEM image) of the plate of 64 titanium after irradiation with a laser beam is presented in FIG. 7.

[Table 1]

|  | Example 1 | Example 2 | Example 3 |
|---|---|---|---|
| Metal plate type | Pure titanium | | 64Ti |
| Thickness of metal plate (mm) | 3.0 | | 1.5 |
| Laser oscillator | Single mode fiber laser | | |
| Output power (W) | 274 | 274 | 250 |
| Wavelength (nm) | 1069 | | |
| Spot diameter ($\mu$m) | 11.25 | | 16.3 |
| Irradiated state | Solid line | Solid line | Solid line |
| Irradiation pattern | Both directions | | |
| Number of lines | 120 | | 120 |
| Treated area (mm$^2$) | 120 | | 120 |
| (a) Irradiation angle | 90 degrees | 90 degrees | 90 degrees |
| (b) Irradiation rate (mm/sec) | 10,000 | | 7500 |
| (c) Energy density (MW/cm$^2$) | 276 | 276 | 119.9 |
| (d) Number of repetitions (times) | 20 | 20 | 10 |
| (e) Defocus distance (mm) | $\pm 0$ | $\pm 0$ | $\pm 0$ |
| (f) Jig (substrate) material | Steel plate | Glass plate | Copper plate |
| (g) Line spacing (b1) (mm) | 0.05 | | |

The size relationship of thermal conductivity (100°C) between pure titanium, 64 Ti, a steel plate, a glass plate, and a copper plate is, in descending order, copper > steel plate > pure titanium > 64 Ti > glass.

When FIG. 5 (Example 1), FIG. 6 (Example 2), and FIG. 7 (Example 3) are compared with one another, biological tissues including bone and teeth in FIG. 6 seem to most readily penetrate into the hole interiors, but the bindability between the bone and the implant is considered ultimately greater in FIGS. 5 and 7 in which the hole structure is more complex.

Examples 4 to 8

Using the same laser device as those in Examples 1 and 2, each of plates (length: 60 mm, width: 10 mm, thickness: 2 mm) of pure titanium was continuously irradiated with a laser beam in area of 5 mm (length) x 10 mm (width) under conditions shown in Table 2.

In Example 8, irradiation was performed in a length direction and in a direction orthogonal thereto of the pure titanium plate. Irradiation was performed in each direction, with 63 lines in the length direction and 125 lines in the orthogonal direction. Specifically, irradiation of the 63 lines was performed 10 times, with a line spacing of 0.08 mm in the length direction. Subsequently, irradiation of the 125 lines was performed 10 times, with a line spacing of 0.08 mm in the orthogonal direction.

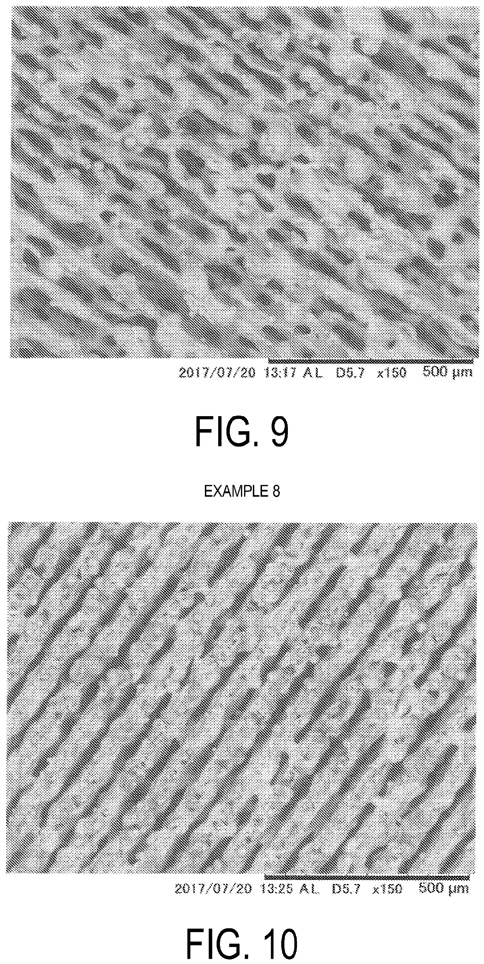

Surface images (SEM images) of the plates of pure titanium after irradiated with a laser beam are presented in FIG. 8 (Example 5), FIG. 9 (Example 7), and FIG. 10 (Example 8).

[Table 2]

|  | Example 4 | Example 5 | Example 6 | Example 7 | Example 8 |
|---|---|---|---|---|---|
| Metal plate type | Pure titanium | | | | |
| Thickness of metal plate (mm) | 2.0 | | | | |
| Laser oscillator | Single mode fiber laser | | | | |
| Output power (W) | 274 | | | | |

(continued)

|  | Example 4 | Example 5 | Example 6 | Example 7 | Example 8 |
|---|---|---|---|---|---|
| Wavelength (nm) | 1069 | | | | |
| Spot diameter ($\mu$m) | 11.25 | | | | |
| Irradiated state | Solid line | | | | |
| Irradiation pattern | Both directions | Both directions | Both directions | Both directions | Intersecting |
| Number of lines | 63 | 63 | 63 | 100 | 63+125 |
| Assist gas (pressure MPa) | None | $N_2$ (0.3) | Ar (0.3) | Ar (0.1) | Ar (0.1) |
| Treated area (mm$^2$) | 50 | | | | |
| (a) Irradiation angle | 90 degrees | | | | |
| (b) Irradiation rate (mm/sec) | 10,000 | | | | |
| (c) Energy density (MW/cm$^2$) | 276 | | | | |
| (d) Number of repetitions (times) | 10 | 10 | 10 | 10 | 10 + 10 |
| (e) Defocus distance (mm) | $\pm 0$ | $\pm 0$ | $\pm 0$ | $\pm 0$ | $\pm 0$ |
| (f) Jig (substrate) material | Steel plate | | | | |
| (g) Line spacing (b1) (mm) | 0.08 | 0.08 | 0.08 | 0.05 | 0.08 |

Examples 9 to 13

Using the same laser device as those described in Examples 1 and 2, each of plates (length: 90 mm, width: 10 mm, thickness: 2 mm) of 64 titanium was continuously irradiated with a laser beam area of 5 mm (length) x 10 mm (width) under conditions shown in Table 3.

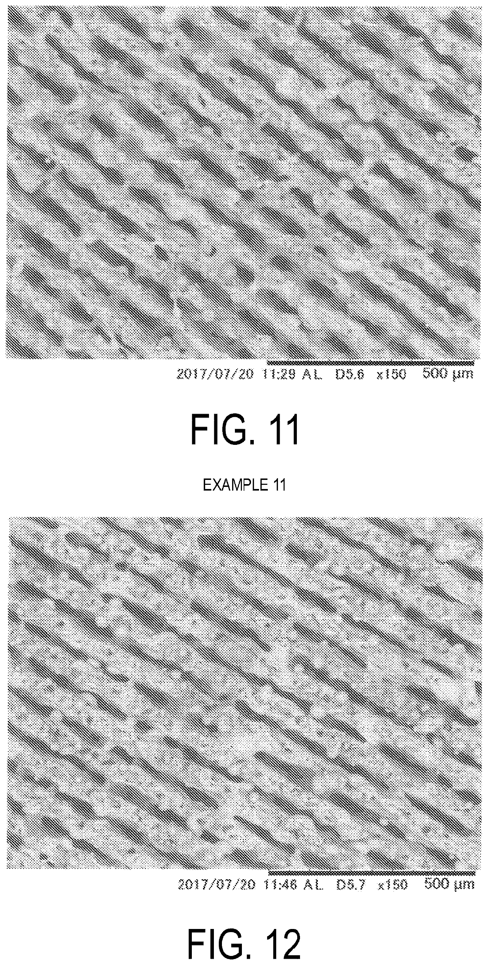

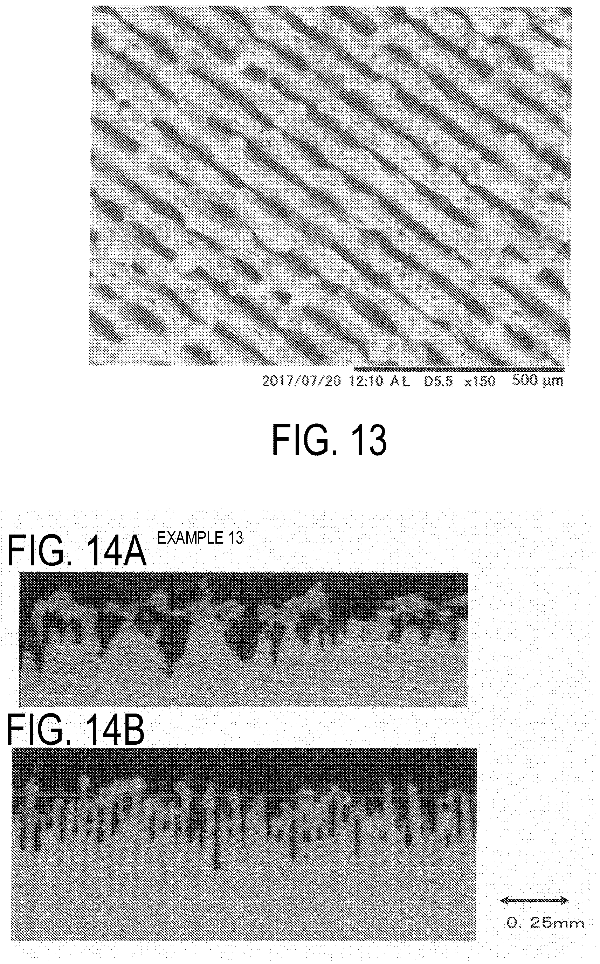

Surface images (SEM images) of the plates of 64 titanium after irradiation with a laser beam are presented in FIG. 11 (Example 9), FIG. 12 (Example 11), and FIG. 13 (Example 12).

Further, x-ray CT scan images of a plate surface of 64 titanium after irradiation with a laser beam in Example 13 are presented in FIG. 14A (a cross sectional image parallel to a scanning direction of the laser beam) and FIG. 14B (a cross-sectional image perpendicular to the scanning direction of the laser beam). The maximum depth of the holes determined from the x-ray CT scan images was 380 $\mu$m.

[Table 3]

|  | Example 9 | Example 10 | Example 11 | Example 12 | Example 13 |
|---|---|---|---|---|---|
| Metal plate type | 64 titanium | | | | |
| Thickness of metal plate (mm) | 2.0 | | | | |
| Laser oscillator | Single mode fiber laser | | | | |
| Output power (W) | 274 | | | | |
| Wavelength (nm) | 1069 | | | | |
| Spot diameter ($\mu$m) | 11.25 | | | | |
| Irradiated state | Solid line | | | | |
| Irradiation pattern | Both directions | | | | |
| Number of lines | 63 | 63 | 63 | 63 | 100 |
| Assist gas (pressure MPa) | None | $N_2$ (0.3) | Ar (0.3) | Ar (0. 1) | Ar (O. 1) |
| Treated area (mm$^2$) | 50 | | | | |
| (a) Irradiation angle | 90 degrees | | | | |

(continued)

|  | Example 9 | Example 10 | Example 11 | Example 12 | Example 13 |
|---|---|---|---|---|---|
| (b) Irradiation rate (mm/sec) | 10,000 | | | | |
| (c) Energy density (MW/cm$^2$) | 276 | | | | |
| (d) Number of repetitions (times) | 10 | 10 | 10 | 20 | 10 |
| (e) Defocus distance (mm) | ±0 | ±0 | ±0 | ±0 | ±0 |
| (f) Jig (substrate) material | Steel plate | | | | |
| (g) Line spacing (b1) (mm) | 0.08 | 0.08 | 0.08 | 0.08 | 0.05 |

Test Example 1

Composition analysis of a pure titanium plate (not irradiated with a laser beam), a pure titanium plate after laser beam irradiation of Examples 4 to 6, the 64 titanium plates (not irradiated with a laser beam), and the 64 titanium plates after laser beam irradiation of Examples 9 to 11 was performed by SEM-EXD. The results are shown in Table 4.

[Table 4]

|  | Untreated | Example 4 | Example 5 | Example 6 | Untreated | Example 9 | Example 10 | Example 11 |
|---|---|---|---|---|---|---|---|---|
| Metal plate type | Pure titanium plate | | | | 64 titanium plate | | | |
| Aluminum (mass%) |  |  |  |  | 6.8 | 5.0 | 5.4 | 6.2 |
| Oxygen (mass%) | 3.0 | 11.2 | 3.4 | 6.0 | 4.0 | 14.8 | 5.9 | 4.5 |

Examples 14 to 18

Using the laser device described below, plates (length: 50 mm, width: 50 mm, thickness: 1.5 mm) of tantalum was continuously irradiated with a laser beam, each in an area of 5 mm (height) x 10 mm (width) under conditions shown in Table 5. No assist gas was used.

Laser Device

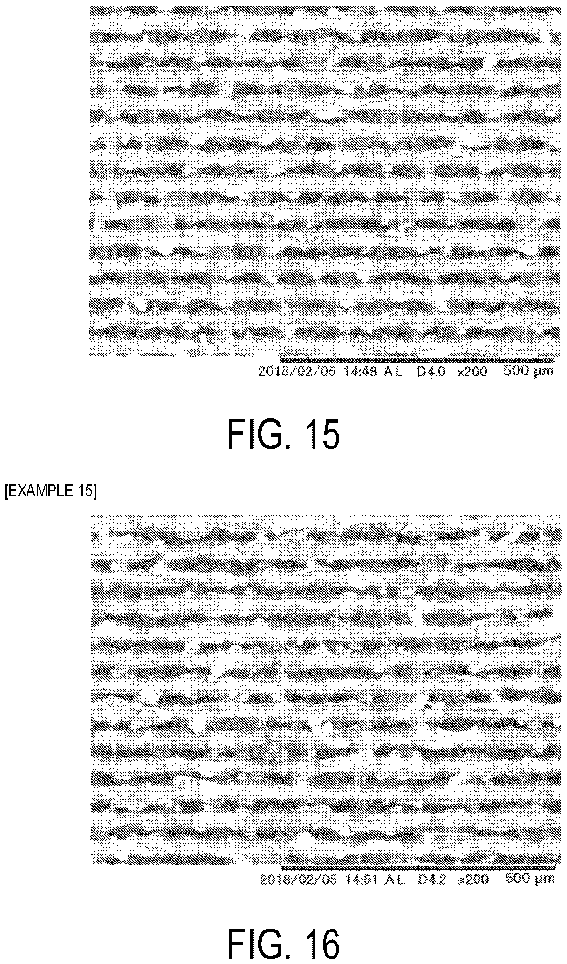

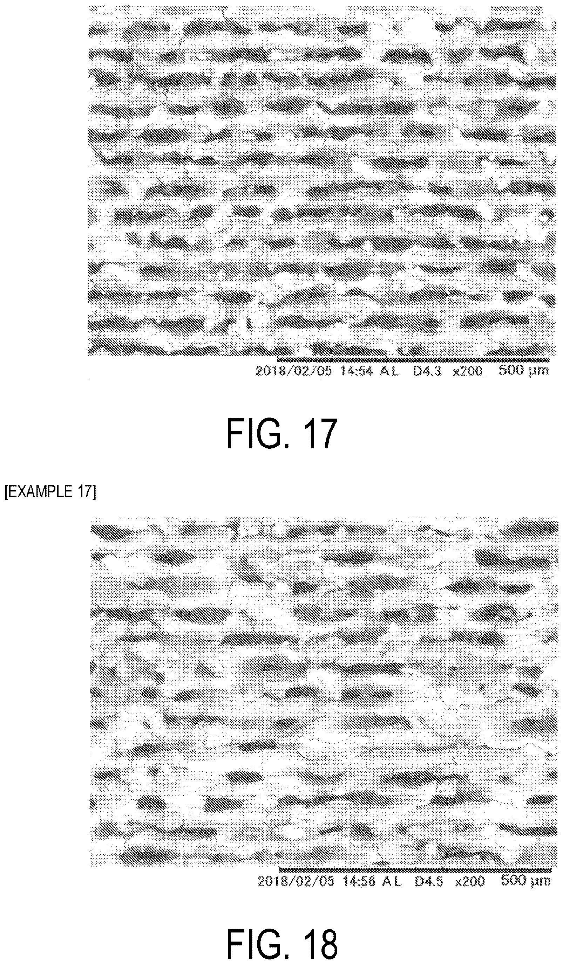

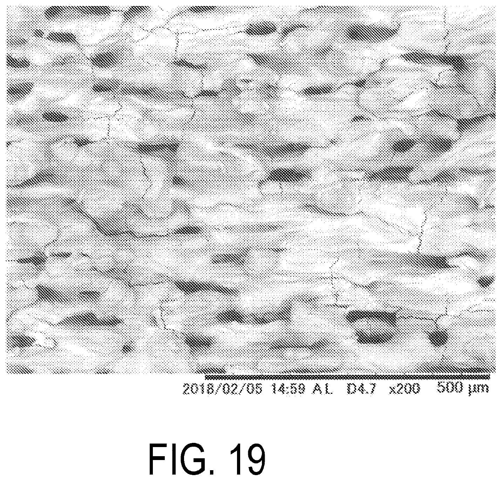

Oscillator: IPG-Yb fiber; YLR-300-AC
Galvano mirror SQUIREEL 16 (available from ARGES)
Light Focusing System: fc = 80 mm/fθ = 100 mm
Surface images (SEM images) of the plates of tantalum after irradiated with a laser beam are presented in FIG. 15 (Example 14), FIG. 16 (Example 15), FIG. 17 (Example 16), FIG. 18 (Example 17), and FIG. 19 (Example 18).

[Table 5]

|  | Example 14 | Example 15 | Example 16 | Example 17 | Example 18 |
|---|---|---|---|---|---|
| Metal plate type | Tantalum | | | | |
| Thickness of metal plate (mm) | 1.5 | | | | |
| Laser oscillator | Single mode fiber laser | | | | |
| Output power (W) | 284 | | | | |
| Wavelength (nm) | 1070 | | | | |

(continued)

|  | Example 14 | Example 15 | Example 16 | Example 17 | Example 18 |
|---|---|---|---|---|---|
| Spot diameter ($\mu$m) | 16.25 | | | | |
| Irradiated state | Solid line | | | | |
| Irradiation pattern | Both directions | | | | |
| Number of lines | 100 | | | | |
| Treated area (mm$^2$) | 50 | | | | |
| (a) Irradiation angle | 90 degrees | | | | |
| (b) Irradiation rate (mm/sec) | 10000 | 7500 | 6000 | 5000 | 4000 |
| (c) Energy density (MW/cm$^2$) | 137 | | | | |
| (d) Number of repetitions (times) | 10 | | | | |
| (e) Defocus distance (mm) | $\pm 0$ | $\pm 0$ | $\pm 0$ | $\pm 0$ | $\pm 0$ |
| (f) Jig (substrate) material | Copper plate | | | | |
| (g) Line spacing (b1) (mm) | 0.05 | | | | |

From the SEM images of Examples 14 to 18, the relationship between the laser irradiation rate and the hole structure can be ascertained.

Industrial Applicability

The implant according to the present invention can be applied to prosthetic joints, such as a prosthetic hip (stem, cup) and a prosthetic knee, implants for fracture fixation (plate, screw), and also to prosthetic tooth roots, and the like.

Reference Signs List

10 Implant
12 Binding face that binds to biological tissue
30 Open hole
31 Opening
32 Trunk hole
33 Branch hole
40 Interior space
45 Open space 45
50 Tunnel connecting path
101 Irradiation portion irradiated by laser beam
102 Non-irradiation portion not irradiated by laser beam

**Claims**

1. An implant (10) for use in a method for binding to a biological tissue including bone or teeth, and made of metal selected from titanium or titanium alloys, cobalt chrome alloys, and tantalum,

the implant (10) including a surface layer portion of a portion, which is bound to a biological tissue including bone or teeth, of the implant (10), the surface layer portion having a porous structure,
the porous structure including
a trunk hole (32) formed in a thickness direction and including an opening(31) on a binding face (12) side,
open holes (30) each constituted of a branch hole (33) formed extending from an inner wall surface of the trunk hole (32) in a direction different from that of the trunk hole (33),
an interior space (40) formed in the thickness direction and not including an opening (31) on the binding face (12) side,
a tunnel connecting path (50) connecting the open holes (30) and the interior space (40), and
a tunnel connecting path (50) connecting the open holes (30).

2. The implant (10) according to claim 1, wherein
the surface layer portion, which has a porous structure, of the implant (10) has a depth ranging from 10 to 1000 $\mu$m from a surface to a depth of the open holes (30).

3. A method for manufacturing an implant (10), the method comprising forming a porous structure in a surface layer portion of the implant (10),

the forming a porous structure in the surface layer portion of the implant (10) including irradiating a surface including the surface layer portion with a laser beam, and
the irradiating with the laser beam including irradiating to alternately generate an irradiation portion (101) irradiated by and a non-irradiation portion (102) not irradiated by the laser beam when irradiating with the laser beam to form a straight line, a curved line, or a combination of the straight line and the curved line,
wherein the implant (10) in an implant for binding to a biological tissue including bone or teeth, and made of metal selected from titanium or titanium alloys, cobalt chrome alloys, and tantalum,
the implant (10) including a surface layer portion of a portion, which is bound to a biological tissue including bone or teeth, of the implant (10), the surface layer portion having a porous structure,
the porous structure including
a trunk hole (32) formed in a thickness direction and including an opening(31) on a binding face (12) side,
open holes (30) each constituted of a branch hole (33) formed extending from an inner wall surface of the trunk hole (32) in a direction different from that of the trunk hole (33),
an interior space (40) formed in the thickness direction and not including an opening (31) on the binding face (12) side,
a tunnel connecting path (50) connecting the open holes (30) and the interior space (40), and
a tunnel connecting path (50) connecting the open holes (30).

4. The method for manufacturing an implant (10) according to claim 3, wherein

the irradiating with a laser beam includes
irradiating with a laser beam by using a combination of a galvano mirror and a galvano controller to pulse, by the galvano controller, a laser beam continuously oscillated from a laser oscillator thereby alternately generating the irradiation portion (101) and the non-irradiation portion (102), or
irradiating with a laser beam by using a fiber laser device provided with a direct-modulation modulator that directly converts a drive current of a laser and that is connected to a laser power supply thereby alternately generating the irradiation portion (101) and the non-irradiation portion (102).

5. The method for manufacturing an implant (10) according to any one of claims 3 to 4, wherein
when performing the irradiating with a laser beam, the irradiation is performed while supplying an assist gas selected from air, oxygen, nitrogen, argon, and helium.

6. The method for manufacturing an implant (10) according to any one of claims 3 to 5, wherein
when performing the irradiating with a laser beam, all of requirements (a) to (g) are adjusted to control a hole orientation, a hole size, and a hole depth:

(a) Irradiation direction and angle of the laser beam
(b) Irradiation rate of the laser beam
(c) Energy density when irradiating with the laser beam
(d) Number of repetitions when irradiating with the laser beam
(e) Defocus distance of the laser beam
(f) Relationship of thermal conductivity between the implant (10) and a substrate, on which the implant (10) is placed, when irradiating with the laser beam
(g) Line spacing of the laser beam.

**7.** The method for manufacturing an implant (10) according to any one of claims 3 to 5, wherein when performing the irradiating with a laser beam, all of requirements (a) to (h) are adjusted to control a hole orientation, a hole size, and a hole depth:

(a) Irradiation direction and angle of the laser beam
(b) Irradiation rate of the laser beam
(c) Energy density when irradiating with the laser beam
(d) Number of repetitions when irradiating with the laser beam
(e) Defocus distance of the laser beam
(f) Relationship of thermal conductivity between the implant (10) and a substrate, on which the implant (10) is placed, when irradiating with the laser beam
(g) Line spacing of the laser beam
(h) Duty ratio: From 30 to 80%.

**8.** The method for manufacturing an implant (10) according to claim 6 or 7, wherein

the irradiation angle of (a) is from 45 to 90 degrees,
the irradiation rate of the laser beam of (b) is from 2000 to 15000 mm/sec,
the energy density when irradiating with the laser beam of (c) is from 2 to 1000 MW/cm$^2$,
the number of repetitions of (d) is from 1 to 40,
the defocus distance of the laser beam of (e) is from -1 to +0.5 mm,
the relationship of thermal conductivity of (f) is as follows: thermal conductivity of implant (10) < thermal conductivity of substrate, and
the line spacing of (g) is from 0.01 to 3 mm.

**9.** The method for manufacturing an implant (10) according to claim 6 or 7, wherein

the irradiation angle of (a) is from 45 to 90 degrees,
the irradiation rate of the laser beam of (b) is from 3000 to 15000 mm/sec,
the energy density when irradiating with the laser beam of (c) is from 100 to 300 MW/cm$^2$,
the number of repetitions of (d) is from 5 to 20,
the defocus distance of the laser beam of (e) is from -0.3 to -0.05 mm,
the relationship of thermal conductivity of (f) is as follows: thermal conductivity of implant (10) < thermal conductivity of substrate, and
the line spacing of (g) is from 0.03 to 0.1 mm.

**Patentansprüche**

**1.** Implantat (10) zur Verwendung in einem Verfahren zum Binden an ein biologisches Gewebe, beinhaltend Knochen oder Zähne, und hergestellt aus Metall, ausgewählt aus Titan oder Titanlegierungen, Kobalt-Chrom-Legierungen und Tantal,

wobei das Implantat (10) einen Oberflächenschichtteil eines Teils, der an ein biologisches Gewebe, beinhaltend Knochen oder Zähne, des Implantats (10) gebunden ist, beinhaltet, wobei der Oberflächenschichtteil eine poröse Struktur besitzt,
wobei die poröse Struktur beinhaltet
ein Stammloch (32), gebildet in einer Dickenrichtung und beinhaltend eine Öffnung (31) auf einer Seite der Bindungsfläche (12),
offene Löcher (30), jeweils bestehend aus einem Abzweigloch (33), gebildet erstreckend von einer Innenwandfläche des Stammlochs (32) in eine Richtung, die sich von derjenigen des Stammlochs (33) unterscheidet,
einen Innenraum (40), gebildet in der Dickenrichtung der und nicht beinhaltend eine Öffnung (31) auf der Seite der Bindungsfläche (12),
einen Tunnelverbindungsweg (50), verbindend die offenen Löcher (30) und den Innenraum (40), und
einen Tunnelverbindungsweg (50), verbindend die offenen Löcher (30).

**2.** Das Implantat (10) nach Anspruch 1, wobei
der Oberflächenschichtteil des Implantats (10), der eine poröse Struktur besitzt, eine Tiefe von 10 bis 1000 $\mu$m von

einer Oberfläche bis zu einer Tiefe der offenen Löcher (30) besitzt.

3. Verfahren zum Herstellen eines Implantats (10), wobei das Verfahren das Bilden einer porösen Struktur in einem Oberflächenschichtteil des Implantats (10) umfasst,

wobei das Bilden einer porösen Struktur in dem Oberflächenschichtteil des Implantats (10) das Bestrahlen einer Oberfläche, beinhaltend den Oberflächenschichtteil, mit einem Laserstrahl beinhaltet, und

wobei das Bestrahlen mit dem Laserstrahl Bestrahlen beinhaltet, um abwechselnd einen Bestrahlungsteil (101), bestrahlt durch den Laserstrahl, und einen Nicht-Bestrahlungsteil (102), nicht bestrahlt durch Laserstrahl, zu erzeugen, wenn mit dem Laserstrahl bestrahlt wird, um eine gerade Linie, eine gekrümmte Linie oder eine Kombination aus der geraden Linie und der gekrümmten Linie zu bilden,

wobei das Implantat (10) ein Implantat zum Binden an ein biologisches Gewebe, beinhaltend Knochen oder Zähne, ist und aus einem Metall hergestellt ist, ausgewählt aus Titan oder Titanlegierungen, Kobalt-Chrom-Legierungen und Tantal,

wobei das Implantat (10) einen Oberflächenschichtteil eines Teils, der an ein biologisches Gewebe, beinhaltend Knochen oder Zähne, des Implantats (10) gebunden ist, beinhaltet, wobei der Oberflächenschichtteil eine poröse Struktur besitzt,

wobei die poröse Struktur beinhaltet

ein Stammloch (32), gebildet in einer Dickenrichtung und beinhaltend eine Öffnung (31) auf einer Seite der Bindungsfläche (12),

offene Löcher (30), jeweils bestehend aus einem Abzweigloch (33), gebildet erstreckend von einer Innenwandfläche des Stammlochs (32) in eine Richtung, die sich von derjenigen des Stammlochs (33) unterscheidet,

einen Innenraum (40), gebildet in der Dickenrichtung der und nicht beinhaltend eine Öffnung (31) auf der Seite der Bindefläche (12),

einen Tunnelverbindungsweg (50), verbindend die offenen Löcher (30) und den Innenraum (40), und

einen Tunnelverbindungsweg (50), verbindend die offenen Löcher (30).

4. Das Verfahren zum Herstellen eines Implantats (10) nach Anspruch 3, wobei das Bestrahlen mit einem Laserstrahl beinhaltet:

Bestrahlen mit einem Laserstrahl unter Verwenden einer Kombination aus einem Galvanospiegel und einer Galvanosteuerung, um durch die Galvanosteuerung einen von einem Laseroszillator kontinuierlich oszillierten Laserstrahl zu pulsieren, wodurch abwechselnd der Bestrahlungsteil (101) und der Nicht-Bestrahlungsteil (102) erzeugt werden, oder

Bestrahlen mit einem Laserstrahl unter Verwenden einer Faserlaservorrichtung, versehen mit einem Direktmodulationsmodulator, der einen Antriebsstrom eines Lasers direkt umwandelt und der mit einer Laserstromversorgung verbunden ist, wodurch abwechselnd der Bestrahlungsteil (101) und der Nicht-Bestrahlungsteil (102) erzeugt werden.

5. Das Verfahren zum Herstellen eines Implantats (10) nach irgendeinem der Ansprüche 3 bis 4, wobei wenn das Bestrahlen mit einem Laserstrahl durchgeführt wird, die Bestrahlung durchgeführt wird, während ein Hilfsgas, ausgewählt aus Luft, Sauerstoff, Stickstoff, Argon und Helium, zugeführt wird.

6. Das Verfahren zum Herstellen eines Implantats (10) nach irgendeinem der Ansprüche 3 bis 5, wobei wenn das Bestrahlen mit einem Laserstrahl durchgeführt wird, alle Anforderungen (a) bis (g) angepasst werden, um eine Ausrichtung, eine Lochgröße und eine Lochtiefe zu steuern:

(a) Bestrahlungsrichtung und Winkel des Laserstrahls
(b) Bestrahlungsrate des Laserstrahls
(c) Energiedichte beim Bestrahlen mit dem Laserstrahl
(d) Anzahl der Wiederholungen beim Bestrahlen mit dem Laserstrahl
(e) Defokussierungsabstand des Laserstrahls
(f) Verhältnis der Wärmeleitfähigkeit zwischen dem Implantat (10) und einem Substrat, auf dem das Implantat (10) angeordnet ist, beim Bestrahlen mit dem Laserstrahl
(g) Linienabstand des Laserstrahls.

7. Das Verfahren zum Herstellen eines Implantats (10) nach irgendeinem der Ansprüche 3 bis 5, wobei wenn das Bestrahlen mit einem Laserstrahl durchgeführt wird, alle Anforderungen (a) bis (h) angepasst werden,

**EP 3 649 986 B1**

um eine Ausrichtung, eine Lochgröße und eine Lochtiefe zu steuern:

(a) Bestrahlungsrichtung und Winkel des Laserstrahls
(b) Bestrahlungsrate des Laserstrahls
(c) Energiedichte beim Bestrahlen mit dem Laserstrahl
(d) Anzahl der Wiederholungen beim Bestrahlen mit dem Laserstrahl
(e) Defokussierungsabstand des Laserstrahls
(f) Verhältnis der Wärmeleitfähigkeit zwischen dem Implantat (10) und einem Substrat, auf dem das Implantat (10) angeordnet ist, beim Bestrahlen mit dem Laserstrahl
(g) Linienabstand des Laserstrahls.
(h) Relative Einschaltdauer (duty ratio): von 30 bis 80%.

8. Das Verfahren zum Herstellen eines Implantats (10) nach Anspruch 6 oder 7, wobei

der Bestrahlungswinkel von (a) zwischen 45 und 90 Grad ist,
die Bestrahlungsrate des Laserstrahls von (b) zwischen 2000 und 15000 mm/s ist,
die Energiedichte beim Bestrahlen mit dem Laserstrahl von (c) zwischen 2 und 1000 MW/cm$^2$ ist,
die Anzahl der Wiederholungen von (d) zwischen 1 und 40 ist,
der Defokussierungsabstand des Laserstrahls von (e) zwischen -1 und +0,5 mm ist,
das Verhältnis der Wärmeleitfähigkeit von (f) wie folgt ist: Wärmeleitfähigkeit des Implantats (10) < Wärmeleitfähigkeit des Substrats, und
der Linienabstand von (g) zwischen 0,01 und 3 mm ist.

9. Das Verfahren zum Herstellen eines Implantats (10) nach Anspruch 6 oder 7, wobei

der Bestrahlungswinkel von (a) zwischen 45 und 90 Grad ist,
die Bestrahlungsrate des Laserstrahls von (b) zwischen 3000 und 15000 mm/s ist,
die Energiedichte beim Bestrahlen mit dem Laserstrahl von (c) zwischen 100 bis 300 MW/cm$^2$ ist,
die Anzahl der Wiederholungen von (d) zwischen 5 und 20 ist,
der Defokussierungsabstand des Laserstrahls von (e) zwischen -0,3 und -0,05 mm ist,
das Verhältnis der Wärmeleitfähigkeit von (f) wie folgt ist: Wärmeleitfähigkeit des Implantats (10) < Wärmeleitfähigkeit des Substrats, und
der Linienabstand von (g) zwischen 0,03 und 0,1 mm ist.

**Revendications**

1. Implant (10) à utiliser dans un procédé de liaison à un tissu biologique comprenant un os ou une dent, et constitué d'un métal choisi parmi le titane ou des alliages de titane, des alliages de chrome-cobalt et le tantale,

l'implant (10) comprenant une partie de couche de surface d'une partie, qui est liée à un tissu biologique comprenant un os ou des dents, de l'implant (10), la partie de couche de surface ayant une structure poreuse, la structure poreuse comprenant
un trou de tronc (32) formé dans une direction d'épaisseur et comprenant une ouverture (31) sur un côté face de liaison (12),
des trous ouverts (30) constitués chacun d'un trou de ramification (33) formé en s'étendant depuis une surface de paroi intérieure du trou de tronc (32) dans une direction différente de celle du trou de tronc (33),
un espace intérieur (40) formé dans la direction d'épaisseur et ne comportant pas d'ouverture (31) sur le côté face de liaison (12),
un chemin de raccordement en tunnel (50) raccordant les trous ouverts (30) et l'espace intérieur (40), et
un chemin de raccordement en tunnel (50) raccordant les trous ouverts (30).

2. Implant (10) selon la revendication 1, dans lequel
la partie de couche de surface, qui a une structure poreuse, de l'implant (10) a une profondeur allant de 10 à 1000 μm depuis une surface jusqu'à une profondeur des trous ouverts (30).

3. Procédé de fabrication d'un implant (10), le procédé comprenant la formation d'une structure poreuse dans une partie de couche de surface de l'implant (10),

la formation d'une structure poreuse dans la partie de couche de surface de l'implant (10) comprenant l'irradiation d'une surface comprenant la partie de couche de surface avec un faisceau laser, et

l'irradiation avec le faisceau laser comprenant l'irradiation pour générer en alternance une partie d'irradiation (101) irradiée par et une partie de non-irradiation (102) non irradiée par le faisceau laser lors de l'irradiation avec le faisceau laser pour former une ligne droite, une ligne incurvée, ou une combinaison de la ligne droite et de la ligne incurvée,

dans lequel l'implant (10) est un implant pour se lier à un tissu biologique comprenant un os ou des dents, et constitué d'un métal choisi parmi le titane ou des alliages de titane, des alliages de chrome-cobalt et le tantale,

l'implant (10) comprenant une partie de couche de surface d'une partie, qui est liée à un tissu biologique comprenant un os ou des dents, de l'implant (10), la partie de couche de surface ayant une structure poreuse, la structure poreuse comprenant

un trou de tronc (32) formé dans une direction d'épaisseur et comprenant une ouverture (31) sur un côté face de liaison (12),

des trous ouverts (30) constitués chacun d'un trou de ramification (33) formé en s'étendant depuis une surface de paroi intérieure du trou de tronc (32) dans une direction différente de celle du trou de tronc (33),

un espace intérieur (40) formé dans la direction d'épaisseur et ne comportant pas d'ouverture (31) sur le côté face de liaison (12),

un chemin de raccordement en tunnel (50) raccordant les trous ouverts (30) et l'espace intérieur (40), et

un chemin de raccordement en tunnel (50) raccordant les trous ouverts (30).

4. Procédé de fabrication d'un implant (10) selon la revendication 3, dans lequel

l'irradiation avec un faisceau laser comprend

l'irradiation avec un faisceau laser en utilisant une combinaison d'un miroir galvano et d'une unité de commande galvano pour pulser, par l'unité de commande galvano, un faisceau laser oscillant en continu à partir d'un oscillateur laser, générant ainsi en alternance la partie d'irradiation (101) et la partie de non-irradiation (102), ou l'irradiation avec un faisceau laser en utilisant un dispositif laser à fibre pourvu d'un modulateur à modulation directe qui convertit directement un courant d'attaque d'un laser et qui est connecté à une alimentation laser générant ainsi en alternance la partie d'irradiation (101) et la partie de non-irradiation (102).

5. Procédé de fabrication d'un implant (10) selon l'une quelconque des revendications 3 et 4, dans lequel lors de la réalisation de l'irradiation avec un faisceau laser, l'irradiation est réalisée tout en fournissant un gaz d'assistance choisi parmi l'air, l'oxygène, l'azote, l'argon et l'hélium.

6. Procédé de fabrication d'un implant (10) selon l'une quelconque des revendications 3 à 5, dans lequel lors de la réalisation de l'irradiation avec un faisceau laser, toutes les exigences (a) à (g) sont ajustées pour commander une orientation de trou, une taille de trou et une profondeur de trou :

(a) une direction d'irradiation et un angle du faisceau laser
(b) une vitesse d'irradiation du faisceau laser
(c) une densité d'énergie lors de l'irradiation avec le faisceau laser
(d) un nombre de répétitions lors de l'irradiation avec le faisceau laser
(e) une distance de défocalisation du faisceau laser
(f) une relation de conductivité thermique entre l'implant (10) et un substrat, sur lequel l'implant (10) est placé, lors de l'irradiation avec le faisceau laser
(g) un espacement des lignes du faisceau laser.

7. Procédé de fabrication d'un implant (10) selon l'une quelconque des revendications 3 à 5, dans lequel lors de la réalisation de l'irradiation avec un faisceau laser, toutes les exigences (a) à (h) sont ajustées pour commander une orientation de trou, une taille de trou et une profondeur de trou :

(a) une direction d'irradiation et un angle du faisceau laser
(b) une vitesse d'irradiation du faisceau laser
(c) une densité d'énergie lors de l'irradiation avec le faisceau laser
(d) un nombre de répétitions lors de l'irradiation avec le faisceau laser
(e) une distance de défocalisation du faisceau laser
(f) une relation de conductivité thermique entre l'implant (10) et un substrat, sur lequel l'implant (10) est placé, lors de l'irradiation avec le faisceau laser

(g) un espacement des lignes du faisceau laser

(h) un rapport cyclique : de 30 à 80%.

8. Procédé de fabrication d'un implant (10) selon la revendication 6 ou 7, dans lequel

l'angle d'irradiation de (a) est de 45 à 90 degrés,
la vitesse d'irradiation du faisceau laser de (b) est de 2000 à 15000 mm/sec,
la densité d'énergie lors de l'irradiation avec le faisceau laser de (c) est de 2 à 1000 MW/cm$^2$,
le nombre de répétitions de (d) est de 1 à 40,
la distance de défocalisation du faisceau laser de (e) est de -1 à +0,5 mm,
la relation de conductivité thermique de (f) est la suivante : la conductivité thermique de l'implant (10) < la conductivité thermique du substrat, et
l'espacement des lignes de (g) est de 0,01 à 3 mm.

9. Procédé de fabrication d'un implant (10) selon la revendication 6 ou 7, dans lequel

l'angle d'irradiation de (a) est de 45 à 90 degrés,
la vitesse d'irradiation du faisceau laser de (b) est de 3000 à 15000 mm/sec,
la densité d'énergie lors de l'irradiation avec le faisceau laser de (c) est de 100 à 300 MW/cm$^2$,
le nombre de répétitions de (d) est de 5 à 20,
la distance de défocalisation du faisceau laser de (e) est de -0,3 à -0,05 mm,
la relation de conductivité thermique de (f) est la suivante : la conductivité thermique de l'implant (10) < la conductivité thermique du substrat, et
l'espacement des lignes de (g) est de 0,03 à 0,1 mm.

FIG. 1A

FIG. 1B

FIG. 2A

FIG. 2B

FIG. 2C

FIG. 3

FIG. 4A

FIG. 4B

FIG. 5

FIG. 6

FIG. 7

EXAMPLE 5

FIG. 8

2017/07/20 13:17 AL D5.7 x150 500 µm

# FIG. 9

EXAMPLE 8

2017/07/20 13:25 AL D5.7 x150 500 µm

# FIG. 10

2017/07/20 11:29 AL D5.6 x150 500 µm

# FIG. 11

EXAMPLE 11

2017/07/20 11:46 AL D5.7 x150 500 µm

# FIG. 12

2017/07/20 12:10 AL D5.5 x150 500 μm

# FIG. 13

FIG. 14A EXAMPLE 13

FIG. 14B

0. 25mm

2018/02/05 14:48 AL D4.0 x200 500 µm

# FIG. 15

[EXAMPLE 15]

2018/02/05 14:51 AL D4.2 x200 500 µm

# FIG. 16

2018/02/05 14:54 AL D4.3 x200 500 μm

# FIG. 17

[EXAMPLE 17]

2018/02/05 14:56 AL D4.5 x200 500 μm

# FIG. 18

2018/02/05 14:59 AL D4.7 x200 500 μm

# FIG. 19

**EP 3 649 986 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5920030 B
- JP 2009254581 A
- JP 2014161520 A
- JP 5326164 B
- WO 2013150369 A1
- JP 5860190 B
- JP 5774246 B
- JP 5701414 B
- JP 5890054 B
- JP 5959689 B
- JP 2016043413 A
- JP 2016036884 A
- JP 2016044337 A
- JP 2016078090 A
- JP 2016124024 A